(19) Europäisches Patentamt
European Patent Office
Office européen des brevets

(11) EP 2 271 302 B1

(12) # EUROPÄISCHE PATENTSCHRIFT

(45) Veröffentlichungstag und Bekanntmachung des Hinweises auf die Patenterteilung:
**12.07.2017 Patentblatt 2017/28**

(21) Anmeldenummer: **09741918.8**

(22) Anmeldetag: **17.02.2009**

(51) Int Cl.:
***A61K 8/34*** (2006.01) ***A61K 8/44*** (2006.01)
***A61K 8/49*** (2006.01) ***A61K 8/86*** (2006.01)
***A61K 8/22*** (2006.01) ***A61Q 5/08*** (2006.01)

(86) Internationale Anmeldenummer:
**PCT/EP2009/051835**

(87) Internationale Veröffentlichungsnummer:
**WO 2009/135700 (12.11.2009 Gazette 2009/46)**

(54) **AUFHELLMITTEL MIT EINEM KATIONISCHEN ACYLPYRIDINIUMDERIVAT, EINEM CO-BLEICHAKTIVATOR UND WASSERSTOFFPEROXID**

BLEACHING AGENT COMPRISING A CATIONIC ACYL PYRIDINIUM DERIVATIVE, A CO-BLEACHING ACTIVATOR, AND HYDROGEN PEROXIDE

AGENT ÉCLAIRCISSANT CONTENANT UN DÉRIVÉ CATIONIQUE D’ACYLPYRIDINIUM , UN CO-ACTIVATEUR DE BLANCHIMENT ET DU PEROXYDE D’HYDROGÈNE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **07.05.2008 DE 102008022710**

(43) Veröffentlichungstag der Anmeldung:
**12.01.2011 Patentblatt 2011/02**

(73) Patentinhaber: **Henkel AG & Co. KGaA**
**40589 Düsseldorf (DE)**

(72) Erfinder:
- **GROß, Wibke**
**41836 Hückelhoven (DE)**
- **FUHR, Denise**
**22559 Hamburg (DE)**
- **NEMITZ, Ralph**
**41363 Jüchen (DE)**
- **MIEHLICH,Kristin geb.Pauli.**
**42119 Wuppertal (DE)**

(56) Entgegenhaltungen:
**EP-A- 1 219 285** **EP-A- 1 882 495**
**EP-A- 1 891 927** **EP-A- 1 905 418**
**DE-A1- 19 745 356** **GB-A- 645 263**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 271 302 B1

Printed by Jouve, 75001 PARIS (FR)

**Beschreibung**

Die vorliegende Erfindung betrifft Mittel zum Aufhellen keratinischer Fasern, d.h. Mittel zur Anwendung auf Keratinfasern, insbesondere menschlichen Haaren, enthaltend kationische Acylpyridiniumderivate, einen toxikologisch unbedenklichem Co-Bleichaktivator und Wasserstoffperoxid zum Aufhellen von Haaren sowie ein entsprechendes Verfahren.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und Blondierverfahren auf bestimmte Ausgangshaarfarben begrenzt. Die Grundlagen der Blondierverfahren sind dem Fachmann bekannt und können in einschlägigen Monographien, z. B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

Neben der Färbung ist das Aufhellen der eigenen Haarfarbe bzw. das Blondieren der ganz spezielle Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalten eingesetzt. Mit der Aufhellung geht jedoch auch eine Schädigung des Haares einher, da nicht nur die natürlichen farbgebenden Komponenten des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Peroxodisulfate ist, desto stärkere Schädigungen werden in der Regel auf der Keratinfaser hervorgerufen. Haarfärbe- bzw. Aufhellmittel, welche eine gute Aufhellleistung zeigen, ohne gleichzeitig die Haarfaser zu schädigen, sind bislang nicht bekannt.

Vor ihrer Anwendung auf menschliches Haar werden Haarfärbe- und/oder -aufhellungsmittel in fester oder pastöser Form üblicherweise mit verdünnter wässriger Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung bzw. Aufhellung liegt zwischen etwa 30 und 40 Minuten. Es ist nahe liegend, dass bei den Benutzern dieser Haarfarben oder Blondiermittel ein Bedürfnis besteht, diese Einwirkungszeit zu verringern.

Blondierprozesse an keratinischen Fasern laufen üblicherweise bei alkalischen pH-Werten ab, insbesondere zwischen 9,0 und 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Als Alkalisierungsmittel wird üblicherweise Ammoniak eingesetzt, der allerdings für den Anwender den Nachteil des intensiven Geruches und eventueller Reizung bis hin zu Hautirritationen und Hautsensibilisierungen aufweist.

Auch wenn die bislang auf dem Markt befindlichen Blondiermittel in der Regel gute Aufhellleistungen zeigen, so können sie aufgrund von Haarschädigung, langen Anwendungszeiten und der aufgrund der hohen Konzentrationen an Oxidations- und Alkalisierungsmittel möglichen Hautreizungen nicht als optimal angesehen werden.

Der Einsatz von kationischen Acylpyridiniumderivaten in der Haarfärbung ist beispielsweise aus den Schriften DE 10148845 A1 oder DE 10261656 A1 bekannt. In beiden Dokumenten werden diese Derivate jedoch zusammen mit mindestens einer zweiten färbenden Komponente als Mittel zur Färbung und damit zur Steigerung der Farbintensität des Haares beschrieben. Aus dem Stand der Technik ist bislang nicht ersichtlich, dass diese 4-Acylpyridiniumderivate in spezieller Kombination mit speziellen, toxikologisch unbedenklichen Co-Bleichaktivatoren und Wasserstoffperoxid für die Bleiche des Haares mit sehr guter Entfärbekraft eingesetzt werden können. Der Einsatz von Sechsring-N-Heterocyclen zur Aktivierung von Wasserstoffperoxid und zur Verbesserung der Aufhellwirkung von Wasserstoffperoxid an keratinischen Fasern ist aus der Schrift EP 1 905 418 A2 bekannt.

Es ist die Aufgabe dieser Erfindung, neuartige Mittel zum Aufhellen bzw. Blondieren von Haaren bereitzustellen, welche in ihrer Aufhellleistung den üblichen auf dem Markt befindlichen Mitteln vergleichbar oder überlegen sind, gleichzeitig jedoch eine verringerte Haarschädigung aufweisen.

In nicht vorhersehbarer Weise konnte nun gefunden werden, dass der Einsatz einer Kombination von kationischen Acylpyridiniumverbindungen der allgemeinen Struktur (I), mindestens einem toxikologisch unbedenklichem Co-Bleichaktivator und Wasserstoffperoxid die Haare viel stärker aufhellt, als es durch den Einsatz einer vergleichbaren

Menge Wasserstoffperoxid allein möglich wäre.

Bedingt durch die verbesserte Blondierleistung bei Anwendung des erfindungsgemäßen Mittels kann die Menge an eingesetztem Oxidationsmittel vermindert und die Haarschädigung hierdurch minimiert werden. Auch eine Verkürzung der Einwirkzeit unter Erzielung eines dem Stand der Technik entsprechenden Aufhelleffekts ist auf diese Weise möglich.

Die erfindungsgemäßen Mittel entfärben den natürlichen Farbstoff Melanin durch Oxidation. Die erfindungsgemäße Wirkstoffkombination bildet ohne die Gegenwart zusätzlicher Farbstoffe/Farbstoffvorprodukte sichtbar keinen Farbstoff in der keratinhaltigen Faser aus. Auch zuvor auf beziehungsweise in der keratinhaltigen Faser befindliche synthetische Farbstoffe können mit Hilfe der erfindungsgemäßen Mittel ausgeblichen werden.

Ein erster Gegenstand der Erfindung ist daher ein Mittel zum Aufhellen von keratinischen Fasern, dadurch gekennzeichnet, dass es in einem kosmetischen Träger

(i) mindestens ein kationisches Acylpyridiniumderivat der Formel (I)

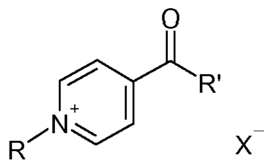

(I)

worin

R für eine $C_1$-$C_6$-Alkylgruppe, eine $C_2$-$C_6$-Alkenylgruppe, oder eine $C_2$-$C_6$-Hydroxyalkylgruppe steht,
R' für eine $C_1$-$C_4$-Alkylgruppe, eine $C_2$-$C_6$-Hydroxyalkylgruppe oder eine $C_1$-$C_6$-Alkoxy-$C_2$-$C_6$-alkylgruppe,
$X^-$ für ein physiologisch verträgliches Anion steht,

(ii) mindestens einen toxikologisch unbedenklichen Co-Bleichaktivator und/oder dessen physiologisch verträgliches Salz, und
(iii) Wasserstoffperoxid enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben und/oder Aufhellen von Keratinfasern geeignet sind, steht prinzipiell einer Verwendung auch auf anderen Gebieten nichts entgegen.

Im Folgenden werden Beispiele für die als Substituenten der Verbindungen der Formel (I) genannten Reste aufgezählt:

Beispiele für $C_1$-$C_6$-Alkylreste sind die Gruppen $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$.

Beispiele für eine $C_2$-$C_6$-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe. Die Prop-2-enylgruppe ist in diesem Zusammenhang besonders bevorzugt.

Weiterhin können als bevorzugte Beispiele für eine $C_2$-$C_6$-Hydroxyalkylgruppe $-CH_2CH_2OH$, $-CH_2CH_2CH_2OH$, $-CH_2CH(OH)CH_3$, $-CH_2CH_2CH_2CH_2OH$ eingesetzt werden, wobei die Gruppe $-CH_2CH_2OH$ bevorzugt ist.

Die erfindungsgemäßen Mittel enthalten mindestens drei wesentliche Bestandteile: mindestens ein kationisches Acylpyridiniumderivat der Formel (I), mindestens einen toxikologisch unbedenklichen Co-Bleichaktivator und/oder dessen physiologisch verträgliches Salz und Wasserstoffperoxid. Erfindungsgemäße Mittel können auch "Anwendungsmischungen" sein, d.h. Mittel, die zwar (z.B. aus Stabilitätsgründen) getrennt verpackt vorliegen, vor der Anwendung aber miteinander zu einer Anwendungsmischung vermischt und dann appliziert werden.

Es ist erfindungsgemäß bevorzugt, wenn der Rest R' der Formel (I) für Methyl, Ethyl, n-Propyl oder Isopropyl steht. Es ist bevorzugt, wenn das Anion $X^-$ gemäß Formel (I) ausgewählt wird aus Halogenid, insbesondere Chlorid, Bromid und Iodid, Benzolsulfonat, p-Toluolsulfonat, $C_1$-$C_4$-Alkylsulfonat, Trifluormethansulfonat, Acetat, Triflluoracetat, Perchlorat, ½ Sulfat, Hydrogensulfat, Tetrafluorborat, Hexafluorphosphat oder Tetrachlorzinkat. Erfindungsgemäß besonders begünstigt ist es, wenn das physiologisch verträgliche Anion $X^-$ für ein Halogenidion (insbesondere Chlorid oder Bromid), Hydrogensulfat, ½ Sulfat, p-Toluolsulfonat, Benzolsulfonat oder Acetat steht.

Besonders bevorzugte kationische Acylpyridiniumderivate der allgemeinen Formel (I) sind

| | | | |
|---|---|---|---|
| Salze des 4-Acetyl-1-methylpyridiniums | 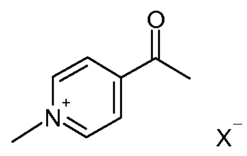 | Salze des 4-Acetyl-1-ethyl-pyridiniums | 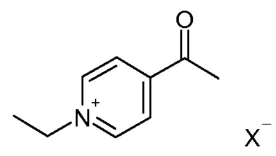 |
| Salze des 4-Acetyl-1-allylpyridiniums | 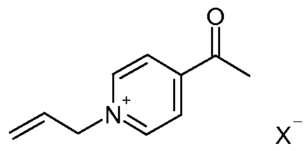 | Salze des 4-Acetyl-1-(2-methylprop-2-enyl)pyridiniums | 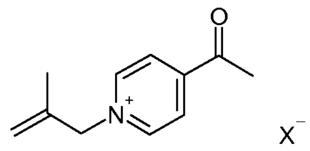 |
| Salze des 4-Acetyl-1-(2-hydroxyethyl)-pyridiniums | 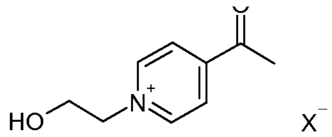 | | |

worin $X^-$ jeweils die Bedeutungen gemäß Struktur (I) bzw. die Bedeutung der vorgenannten bevorzugten Ausführungsformen annimmt.

Zusammenfassend sind erfindungsgemäße Mittel bevorzugt, die als kationisches Acylpyridiniumderivat der allgemeinen Struktur (I) mindestens eine Verbindung aus der Gruppe enthalten, die gebildet wird aus 4-Acetyl-1-methylpyridinium-p-toluolsulfonat, 4-Acetyl-1-methylpyridiniumbenzolsulfonat, 4-Acetyl-1-methylpyridiniumbromid, 4-Acetyl-1-methylpyridiniumhydrogensulfat, 4-Acetyl-1-allyl-pyridinium-p-toluosulfonat, 4-Acetyl-1-allylpyridiniumbenzolsulfonat, 4-Acetyl-1-allylpyridinium-bromid, 4-Acetyl-1-allylpyridiniumhydrogensulfat, 4-Acetyl-1-(2-hydroxyethyl)pyridinium-p-toluolsulfonat, 4-Acetyl-1-(2-hydroxyethyl)pyridiniumbenzolsulfonat, 4-Acetyl-1-(2-hydroxyethyl)-pyridiniumbromid, 4-Acetyl-1-(2-hydroxyethyl)pyridiniumhydrogensulfat, 4-Acetyl-1-ethyl-pyridinium-p-toluolsulfonat, 4-Acetyl-1-ethylpyridiniumbenzolsulfonat, 4-Acetyl-1-ethylpyridinium-bromid, 4-Acetyl-1-ethylpyridiniumhydrogensulfat, 4-Acetyl-1 -(2-methyl-prop-2-enyl)pyridinium-p-toluolsulfonat, 4-Acetyl-1-(2-methyl-prop-2-enyl)pyridiniumbenzolsulfonat, 4-Acetyl-1-(2-methyl-prop-2-enyl)pyridiniumbromid, 4-Acetyl-1-(2-methyl-prop-2-enyl)pyridiniumhydrogensulfat. Aus dieser Gruppe sind die folgenden Acetylpyridiniumsalze explizit ganz besonders bevorzugt: 4-Acetyl-1-methylpyridinium-p-toluolsulfonat, 4-Acetyl-1-methylpyridiniumbenzolsulfonat, 4-Acetyl-1-methylpyridiniumbromid, 4-Acetyl-1-methylpyridiniumhydrogensulfat, 4-Acetyl-1-allyl-pyridinium-p-toluosulfonat, 4-Acetyl-1-allylpyridiniumbenzolsulfonat, 4-Acetyl-1-allylpyridinium-bromid, 4-Acetyl-1-allylpyridiniumhydrogensulfat, 4-Acetyl-1-(2-hydroxyethyl)pyridinium-p-toluolsulfonat, 4-Acetyl-1-(2-hydroxyethyl)pyridiniumbenzolsulfonat, 4-Acetyl-1-(2-hydroxyethyl)-pyridiniumbromid, 4-Acetyl-1-(2-hydroxyethyl)pyridiniumhydrogensulfat.

Soweit nicht explizit anders angegeben, beziehen sich alle nachfolgend genannten Mengenangaben jeweils auf das Gesamtgewicht des anwendungsbereiten Mittels.

Als ersten wesentlichen Inhaltstoff enthalten die erfindungsgemäßen Mittel die Acylpyridiniumderivate der allgemeinen Struktur (I) bevorzugt in einer Menge von 0,01 bis 25 Gew.-%, insbesondere von 0,1 bis 10 Gew.-%, jeweils bezogen auf das Gesamtgewicht des anwendungsbereiten Mittels.

Als zweiter wesentlicher Inhaltsstoff des erfindungsgemäßen Mittels sind mindestens ein toxikologisch unbedenklichen Co-Bleichaktivator und/oder dessen physiologisch verträgliches Salz enthalten. Als toxikologisch unbedenklich im Sinne der vorliegenden Erfindung ist insbesondere nicht Imidazol zu verstehen.

Dieser toxikologisch unbedenkliche Co-Bleichaktivator ist bevorzugt ausgewählt aus aliphatischen und/oder carbocyclischen Co-Bleichaktivatoren.

Besonders bevorzugt enthält dieser toxikologisch unbedenkliche Co-Bleichaktivator als wesentliches Strukturmerkmal eine Hydroxygruppe, eine Carbonsäure, einen Schwefelsäuremonoester, einen Phosphorsäuremonoester und/oder ein physiologisch verträgliches Salz davon.

Für den Fall, dass es sich der toxikologisch unbedenkliche Co-Bleichaktivator eine Struktureinheit enthält, die mehrere räumliche Anordnungen erlaubt, wie beispielsweise substituierte Doppelbindungen oder Asymmetriezentren, so sind im Rahmen der vorliegenden Erfindung selbstverständlich alle möglichen Stereoisomeren umfasst. Gegebenfalls kann es aber erfindungsgemäß bevorzugt sein, entweder nur ein mögliches Stereoisomer oder aber explizit ein Gemisch aus zwei oder mehreren Stereoisomeren einzusetzen.

Erfindungsgemäß bevorzugte Mittel sind dadurch gekennzeichnet, dass als Co-Bleichaktivator und/oder dessen physiologisch verträgliches Salz mindestens einen Co-Bleichaktivator gemäß Formel (II) und/oder dessen physiologisch verträgliches Salz enthalten ist,

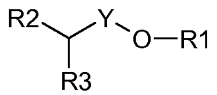

(II)

worin

Y für eine Carbonyl-Gruppe, eine direkte Bindung oder Methylen-Gruppe steht,
R1 für Wasserstoff, eine $C_1$-$C_4$-Alkylgruppe, ein physiologisch verträgliches Kation oder eine $SO_3^-$- oder eine $PO_3^{2-}$- Gruppe steht,
R2 für eine Amino-, eine Methylamino-, eine Dimethylamino-, eine Trimethylammonio-Gruppe, Phenyl, Benzyl, Phenoxymethyl, 1-Naphthyl, 2-Naphthyl, 2-, 3-, 4-Toluolyl, oder eine R4-O-$(CH_2CH_2O)_n$-Gruppe steht, worin R4 für eine $C_6$-$C_{20}$-Alkylgruppe und n für eine Zahl von größer als 15 steht,
R3 für Wasserstoff oder eine gegebenenfalls verzweigte $C_1$-$C_6$-Alkylgruppe steht, mit der Massgabe, dass,

wenn Y für eine Carbonyl-Gruppe steht,
R1 für Wasserstoff, eine $C_1$-$C_4$-Alkylgruppe oder ein physiologisch verträgliches Kation steht,
R2 für eine Amino-, eine Methylamino-, eine Dimethylamino- oder eine Trimethylammonio-Gruppe steht, und

R3 für Wasserstoff oder eine gegebenenfalls verzweigte $C_1$-$C_6$-Alkylgruppe steht, dass,
wenn Y für eine direkte Bindung steht,
R1 für Wasserstoff und
R2 für Phenyl, Benzyl, Phenoxymethyl, 1-Naphthyl, 2-Naphthyl, 2-, 3- oder 4-Toluolyl steht, und
R3 für Wasserstoff oder eine gegebenenfalls verzweigte $C_1$-$C_6$-Alkylgruppe steht,

und dass,

wenn Y für eine Methylen-Gruppe steht,
R1 für eine $SO_3^-$- oder eine $PO_3^{2-}$-Gruppe steht,
R2 für eine R4-$O(CH_2CH_2O)_n$-Gruppe steht, worin R4 für eine $C_6$-$C_{20}$-Alkylgruppe und n für eine Zahl von größer als 15 steht, und
R3 für Wasserstoff steht.

Insbesondere sind erfindungsgemäß bevorzugte Mittel dadurch gekennzeichnet, dass als Co-Bleichaktivator mindestens eine gegebenenfalls an ihren Stickstoffatom N-methylierte oder N,N-dimethylierte, aliphatische Aminosäure und/oder dessen physiologisch verträgliches Salz enthalten ist.

Bevorzugte Co-Bleichaktivatoren sind ausgewählt aus Glycin, N-Methyl-glycin, N,N-Dimethyl-glycin, Alanin, N-Methyl-alanin, N,N-Dimethyl-alanin, Leucin, N-Methyl-leucin, N,N-Dimethyl-leucin, Isoleucin, N-Methyl-isoleucin, N,N-Dimethyl-isoleucin oder deren physiologisch verträglichen Salzen.

Ganz besonders bevorzugt ist im erfindungsgemäßen Mittel als Co-Bleichaktivator Glycin und/oder dessen physiologisch verträgliches Salz enthalten.

Bevorzugte erfindungsgemäße Mittel enthalten als Co-Bleichaktivator mindestens einen aromatischen Alkohol und/oder dessen physiologisch verträgliches Salz.

Als erfindungsgemäß bevorzugte, aromatische Alkohole sind Benzylalkohol, 2-Phenylethylalkohol, 1-Phenylethylalkohol, 2-Phenoxyethanol, 1-Hydroxymethyl-naphthalin und/oder 2-Hydroxymethylnaphthalin zu nennen.

Ein erfindungsgemäß ganz besonders bevorzugter aromatischer Alkohol als Co-Bleichaktivator ist Benzylalkohol.

Schließlich können solche Mittel erfindungsgemäß bevorzugt sein, die als Co-Bleichaktivator ein physiologisch verträgliches Salz eines Alkylethersulfats gemäß Formel (III)

$$R4\text{-}O(CH_2CH_2O)_mSO_3Y \qquad (III)$$

enthalten, worin R4 für eine $C_6$-$C_{20}$-Alkylgruppe und m für eine Zahl von größer als 15 steht und Y für ein Alkali- und/oder Erdalkalimetall, Ammonium, Alkylammonium oder Alkanolammonium steht.

Alkylethersulfate ("Ethersulfate") werden großtechnisch durch $SO_3$- oder Chlorsulfonsäure (CSA)-Sulfatierung von Fettalkohol- oder Oxoalkoholpolyglycolethern und nachfolgende Neutralisation hergestellt. Erfindungsgemäß bevorzugte Beispiele sind die Sulfate in Form ihrer Natrium- und/oder Magnesiumsalze von hoch ethoxylierten Anlagerungsprodukten von mindestens 16, durchschnittlich jedoch 20 bis 40 und insbesondere 25 bis 35 Mol Ethylenoxid (ausgedrückt durch m in Formel (III)) an Capronalkohol, Caprylalkohol, 2-Ethylhexylalkohol, Caprinalkohol, Laurylalkohol, Isotridecylalkohol, Myristylalkohol, Cetylalkohol, Stearylalkohol, Isostearylalkohol, Eicosylalkohol oder deren technische Mischungen. Diese fallen beispielsweise bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen an. Bevorzugt sind technische Fettalkohole mit 12 bis 18 Kohlenstoffatomen, wie beispielsweise Kokos-, Palm-, Palmkern- oder Talgfettalkohol. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 25 bis 35 Mol Ethylenoxid an technische $C_{12/14}$- bzw. $C_{12/18}$-Kokosfettalkoholfraktionen in Form ihrer Natrium- und/oder Magnesiumsalze.

Ein besonders bevorzugter Co-Bleichaktivator ist unter der INCI-Bezeichnung Sodium Coceth-30 sulfate geführt und wird unter dem Handelsnamen Disponil® FES 77 als 31-33 gew.-%ige, wässrige Lösung von der Firma Cognis vertrieben.

Vorzugsweise wird/werden der bzw. die Co-Bleichaktivator(en) innerhalb bestimmter Mengenbereiche eingesetzt. Es sind erfindungsgemäße Mittel bevorzugt, die 0,01 bis 10 Gew.-% und insbesondere 0,1 bis 5 Gew.-%, jeweils bezogen auf das Gesamtgewicht des anwendungsbereiten Mittels, mindestens eines toxikologisch unbedenklichen Co-Bleichaktivators enthalten.

Als dritter wesentlicher Inhaltsstoff ist Wasserstoffperoxid in dem erfindungsgemäßen Mittel enthalten. Bevorzugt wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon $H_2O_2$ (n ist eine positive ganze Zahl größer 0), Harnstoffperoxid und Melaminperoxid, eingesetzt werden. Im letztgenannten Fall setzen die Anlagerungsverbindungen in der erfindungsgemäßen Anwendungsmischung Wasserstoffperoxid frei, d. h. diese Mittel enthalten neben der Anlagerungsverbindung im kosmetischen Träger freies Wasserstoffperoxid.

Erfindungsgemäß ganz besonders bevorzugt wird das Wasserstoffperoxid dem erfindungsgemäßen Mittel als wässrige Wasserstoffperoxid-Lösung zudosiert. Die Konzentration einer Wasserstoffperoxid-Lösung wird einerseits von den gesetzlichen Vorgaben und andererseits von dem gewünschten Effekt bestimmt; vorzugsweise werden 6 bis 12 gew.-%ige Lösungen in Wasser verwendet. Erfindungsgemäß bevorzugte Mittel sind dadurch gekennzeichnet, dass sie - bezogen auf ihr Gesamtgewicht - 0,01 bis 12 Gew.-%, vorzugsweise 0,1 bis 10 Gew.-%, besonders bevorzugt 1 bis 6 Gew.-% Wasserstoffperoxid (berechnet als 100%iges $H_2O_2$) enthalten.

Unter Berücksichtigung der bisher genannten bevorzugten Ausführungsformen stellt es eine ganz spezielle und ausdrücklich bevorzugte Ausführungsform dar, wenn das Mittel zum Aufhellen von keratinischen Fasern in einem kosmetischen Träger als erste Komponente mindestens eine Verbindung ausgewählt aus der Gruppe, die gebildet wird aus 4-Acetyl-1-methylpyridinium-p-toluolsulfonat, 4-Acetyl-1-methylpyridiniumbenzolsulfonat, 4-Acetyl-1-methylpyridiniumbromid, 4-Acetyl-1-methylpyridiniumhydrogensulfat, 4-Acetyl-1-allyl-pyridinium-p-toluosulfonat, 4-Acetyl-1-allylpyridiniumbenzolsulfonat, 4-Acetyl-1-allylpyridinium-bromid, 4-Acetyl-1-allylpyridiniumhydrogensulfat, 4-Acetyl-1-(2-hydroxyethyl)pyridinium-p-toluolsulfonat, 4-Acetyl-1-(2-hydroxyethyl)pyridiniumbenzolsulfonat, 4-Acetyl-1-(2-hydroxyethyl)-pyridiniumbromid und 4-Acetyl-1-(2-hydroxyethyl)pyridiniumhydrogensulfat.

enthält, als zweite Co-Bleichaktivator-Komponente mindestens eine Verbindung ausgewählt aus der Gruppe, die gebildet wird aus Glycin, Benzylalkohol und Sodium Coceth-30 sulfate, und als dritte Komponente Wasserstoffperoxid in den bereits beschriebenen bevorzugten Mengenanteilen enthält.

Ganz besonders bevorzugt sind schließlich Mittel, die eine der nachfolgenden Kombinationen enthalten, worin sich die Gewichtsangaben wiederum auf das Gesamtgewicht des anwendungsbereiten Mittels beziehen:

Kombination (a):

0,1 bis 4,0 Gew.-% 4-Acetyl-1-methylpyridinium-p-toluolsulfonat, 0,1 bis 3,0 Gew.-% Glycin und 0,1 bis 12,0 Gew.-% Wasserstoffperoxid.

Kombination (b):

0,1 bis 4,0 Gew.-% 4-Acetyl-1-methylpyridinium-p-toluolsulfonat, 0,1 bis 3,0 Gew.-% Benzylalkohol und 0,1 bis 12,0 Gew.-% Wasserstoffperoxid.

Kombination (c):

0,1 bis 4,0 Gew.-% 4-Acetyl-1-methylpyridinium-p-toluolsulfonat, 0,1 bis 3,0 Gew.-% Sodium Coceth-30 sulfate (Aktivsubstanz) und 0,1 bis 12,0 Gew.-% Wasserstoffperoxid.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des verwendungsbereiten Mittels zwischen 7 und 11, insbesondere zwischen 8 und 10,5, liegt.Bei den pH-Werten im Sinne der vorliegenden Erfindung handelt es sich um pH-Werte, die bei einer Temperatur von 22 °C gemessen wurden.Die zur Einstellung des bevorzugten pH-Wertes erfindungsgemäß verwendbaren Alkalisierungsmittel können aus der Gruppe, die gebildet wird aus Ammoniak, Alkalimetallhydroxiden, Alkanolaminen, Alkalimetallmetasilikaten, Alkalimetallphosphaten und Alkalimetallhydrogenphosphaten ausgewählt werden.Als Alkalimetallionen dienen bevorzugt Lithium, Natrium, Kalium, insbesondere Natrium oder Kalium.Die als erfindungsgemäßes Alkalisierungsmittel einsetzbaren Alkalimetallhydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Amino-butan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und Persulfaten bzw. Peroxodisulfaten eingesetzt. Es hat sich gezeigt, dass die Beimengung der erfindungsgemäßen Acylpyridiniumderivate der allgemeinen Struktur (I) und toxikologisch unbedenklichen Co-Bleichaktivator nicht nur bei Wasserstoffperoxid allein, sondern auch bei einer Kombination aus Wasserstoffperoxid und Persulfatsalzen bzw. Peroxodisulfatsalzen in einer Steigerung des Aufhellvermögens resultiert.

Daher kann es, sollte der Verbraucher den Wunsch nach einer sehr starken Blondierung verspüren, in einer weiteren Ausführungsform bevorzugt sein, wenn neben der kationischen Acylpyridiniumverbindung der allgemeinen Struktur (I), einem toxikologisch unbedenklichen Co-Bleichaktivator und Wasserstoffperoxid zusätzlich mindestens ein anorganisches Persulfatsalz bzw. Peroxodisulfatsalz in dem Mittel zum Aufhellen der keratinischen Fasern enthalten ist.

Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden.

Ein üblicher Weg besteht daher darin, ein erstes Mittel, welches mindestens ein kationisches Acylpyridiniumderivat der allgemeinen Formel (I) und mindestens einen toxikologisch unbedenklichen Co-Bleichaktivator enthält, direkt vor der Anwendung mit einem zweiten Mittel, in welchem das oder die erfindungsgemäßen Oxidationsmittel enthalten sind, zu vermischen.

Ein weiterer Gegenstand der vorliegenden Beschreibung ist daher ein Mittel zum Aufhellen keratinischer Fasern, insbesondere menschlicher Haare, welches unmittelbar vor dem Aufbringen auf das Haar aus einer fließfähigen Zubereitung A, das die kationischen Acylpyridiniumderivate der allgemeinen Formel (I) und einen toxikologisch unbedenklichen Co-Bleichaktivator enthält und einer Oxidationsmittelzubereitung B, enthaltend mindestens ein Oxidationsmittel, ausgewählt aus Wasserstoffperoxid und/oder dessen Anlagerungsverbindungen an organische oder anorganische Verbindungen, erhalten wird.

Die Oxidationsmittelzubereitung B ist vorzugsweise eine wässrige, fließfähige Oxidationsmittelzubereitung. Dabei sind bevorzugte Mittel zum Aufhellen keratinischer Fasern dadurch gekennzeichnet, dass die fließfähige Oxidationsmittelzubereitung B - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Der Einsatz von Persulfatsalzen bzw. Peroxodisulfatsalzen erfolgt in der Regel in Form eines gegebenenfalls entstaubten Pulvers oder eines in Form gepressten Formkörpers. Um einen vorzeitigen Abbau der Acylpyridiniumderivate durch den Kontakt mit den Persulfaten bzw. Peroxodisulfaten zu vermeiden, ist es bevorzugt, die Persulfate bzw. Peroxodisulfate als separat verpackte Komponente C zur Verfügung zu stellen.

In diesem Zusammenhang sei ein aus 3 Komponenten bestehendes Mittel zum Aufhellen von menschlichen Haaren beschrieben. Dieses Mittel wird unmittelbar vor dem Aufbringen auf das Haar durch sorgfältiges Vermischen einer fließfähigen Zubereitung A, die die kationischen Acylpyridiniumderivate der allgemeinen Formel (I) und einen toxikologisch unbedenklichen Co-Bleichaktivator enthält, einer Oxidationsmittelzubereitung B, enthaltend mindestens ein Oxidationsmittel, ausgewählt aus Wasserstoffperoxid und/oder dessen Anlagerungsverbindungen an organische oder anorganische Verbindungen und zusätzlich einer dritten, in Pulverform vorliegenden Zubereitung C, welche mindestens ein anorganisches Persulfatsalz bzw. Peroxodisulfatsalz enthält, hergestellt.

Die Mischung der Zubereitungen A und B bzw. gegebenenfalls der Zubereitungen A, B und C vor der Anwendung führt zu einer Anwendungsmischung, die ein erfindungsgemäßes Mittel mit den drei zwingenden Inhaltsstoffen ist.

Vorzugsweise wird den fließfähigen Zubereitungen A und/oder B ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren (Seifen), Ethercarbonsäuren der Formel $RO(CH_2CH_2P)_xCH_2COOH$, Acylsarcoside, Acyltauride, Acylisethionate, gegebenenfalls polyalkoxylierte Sulfobernsteinsäuremono- und -dialkylester, lineare Alkansulfonate, lineare $\alpha$-Olefinsulfonate, Sulfonate ungesättigter Fettsäuren, $\alpha$-Sulfofettsäuremethylester, Alkylsulfate und Alkylethersulfate der Formel $RO(CH_2CH_2O)_xSO_3H$, Gemische oberflächenaktiver Hydroxysulfonate, sulfatierte Hydroxyalkylpolyethylen- und/oder Hydroxyalkylenpropylenglykolether, Ester der Weinsäure und Zitronensäure mit Alkoholen, Alkyl- und/oder Alkenyletherphosphate, sulfatierte Fettsäurealkylenglykolester der Formel $RC(O)O(alkO)_nSO_3H$ sowie Monoglyceridsulfate und Monoglyceridethersulfate.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine -COOH- oder -$SO_3H$-Grupp enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das $C_{12}$-$C_{18}$-Acylsarcosin.

Weiterhin hat es sich als vorteilhaft erwiesen, wenn die erfindungsgemäßen Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder $C_2$-$C_6$-Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- $C_{12}$-$C_{30}$-Fettsäuremono- und -diester von Anlagerungsprodukten von 1 bis 30 Mol Ethylenoxid an Glycerin,
- Polyglycerinester und alkoxylierte Polyglycerinester,.
- Anlagerungsprodukte von 5 bis 60 Mol Ethylenoxid an Rizinusöl und gehärtetes Rizinusöl,
- Polyolfettsäureester,
- - alkoxylierte, bevorzugt propoxylierte und insbesondere ethoxylierte, Mono-, Di- und Triglyceride, wie beispielsweise Glycerinmonolaurat + 20 Ethylenoxid und Glycerinmonostearat + 20 Ethylenoxid,
- alkoxylierte Fettsäurealkylester der Formel $RC(O)$-$(OCH_2CH_2)_wOR'$, in der RC(O)- für einen linearen oder verzweigten, gesättigten und/oder ungesättigten Acylrest mit 6 bis 22 Kohlenstoffatomen, R' für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate, Sorbitanmonolaurat und Sorbitanmonolaurat + 20 Mol Ethylenoxid (EO),
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylphenole und Alkylphenolalkoxylate mit 6 bis 21, insbesondere 6 bis 15 Kohlenstoffatomen in der Alkylkette und 0 bis 30 Ethylenoxid- und/oder Propylenoxid-Einheiten. Bevorzugte Vertreter dieser Klasse sind beispielsweise Nonylphenol + 4 EO, Nonylphenol + 9 EO, Octylphenol + 3 EO und Octylphenol + 8 EO;
- Alkylpolyglykoside entsprechend der allgemeinen Formel $RO$-$(Z)_x$, wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten.

Als nichtionische Tenside eignen sich insbesondere $C_8$-$C_{22}$-Alkylmono- und oligoglycoside und deren ethoxylierte Analoga. Insbesondere die nichtethoxylierten Verbindungen haben sich als besonders geeignet erwiesen.

Diese Verbindungen sind dadurch gekennzeichnet, dass als Zuckerbaustein Z beliebige Mono- oder Oligosaccharide eingesetzt werden können. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar.

Die Alkylamidoamine werden üblicherweise durch Amidierung natürlicher oder synthetischer Fettsäuren und Fettsäureschnitte mit Dialkylaminoaminen hergestellt und zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

In einer weiteren bevorzugten Ausführungsform kann die Wirkung des erfindungsgemäßen Wirkstoffes durch Emulgatoren gesteigert werden. Solche Emulgatoren sind beispielsweise

- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,

- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten Fettsäuren mit 8 bis 22 C-Atomen,
- Sterine, insbsondere Zoosterine (Cholesterin und Lanosterin), Phytosterine (Ergosterin, Stigmasterin und Sitosterin) und Mykosterine.
- Phospholipide, z. B. als Lecithine bzw. Phosphatidylcholine,
- Fettsäureester von Zuckern und Zuckeralkoholen, wie Sorbit
- Polyglycerine und Polyglycerinderivate wie beispielsweise Polyglycerinpoly-12-hydroxy-stearat
- Lineare und verzweigte Fettsäuren mit 8 bis 30 C-Atomen und deren Na-, K-, Ammonium-, Ca-, Mg- und Zn- Salze.

Die erfindungsgemäßen Mittel enthalten die Emulgatoren bevorzugt in Mengen von 0,1 bis 25 Gew.-%, insbesondere 0,5 bis 15 Gew.-%, bezogen auf die Gesamtmenge des anwendungsbereiten Mittel.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M. Regitz), 10. Auflage, Georg Thieme Verlag Stuttgart, New York, (1997), Seite 1764, aufgeführten Definitionen enthalten. Nichtionogene Emulgatoren mit einem HLB-Wert von 10-15 können erfindungsgemäß besonders bevorzugt sein.

Unter den genannten Emulgatoren-Typen können die Emulgatoren, welche kein Ethylenoxid und/oder Propylenoxid im Molekül enthalten ganz besonders bevorzugt sein.

Die erfindungsgemäßen Mittel können zusätzlich auch Farbstoffe und/oder Farbstoffvorprodukte enthalten und somit als Mittel bereitgestellt werden, die gleichzeitig aufhellend und färbend wirken. Solche Mittel werden nachfolgend als "Färbemittel", als "aufhellende Färbemittel" oder als "Färbe- und Aufhellmittel" bezeichnet.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder in situ geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, Pyranose-Oxidase (mit z.B. D-Glucose oder Galactose), Glucose-Oxidase (mit D-Glucose), Glycerin-Oxidase (mit Glycerin), Pyruvat-Oxidase (mit Benztraubensäure oder deren Salzen), Alkohol-Oxidase (mit Alkohol wie MeOH, EtOH), Lactat-Oxidase (mit Milchsäure), Tyrosinase-Oxidase (mit (Tyrosin), Uricase (mit Harnsäure), Cholinoxidase (mit Cholin) und Aminosäure-Oxidase (mit Aminosäuren).

Bei einer Anwendung von zusätzlichen Oxidationsmitteln wird das eigentliche Aufhell- und/oder Färbemittel zweckmäßigerweise unmittelbar vor der Anwendung durch Mischung der Zubereitung des zusätzlichen Oxidationsmittels mit der erfindungsgemäßen Wasserstoffperoxid-Lösung und mit der Zubereitung, enthaltend die Verbindungen der Formel (I) und den toxikologisch unbedenklichen Co-Bleichaktivator sowie gegebenenfalls Farbstoffvorprodukte, hergestellt. Das dabei entstehende gebrauchsfertige Aufhell- und/oder Haarfärbepräparat sollte bevorzugt einen pH-Wert im Bereich von 6 bis 12 aufweisen. Besonders bevorzugt ist die Anwendung der Aufhell- und/oder Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente, vermischt mit der Zubereitung, enthaltend Acylpyridiniumderivat gemäß Formel (I) und den toxikologisch unbedenklichen Co-Bleichaktivator, aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst

eine Luftoxidation angestrebt, wobei das aufgebrachte Mittel bevorzugt einen pH-Wert von 7 bis 10 aufweist. Bei der anschließenden beschleunigten Nachoxidation kann die zusätzliche Verwendung von sauer eingestellten Peroxodisulfat-Lösungen als Oxidationsmittel bevorzugt sein.

Ein Einsatz bestimmter Metallionen oder -komplexe kann ebenfalls bevorzugt sein, um intensive Färbungen zu erhalten. Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$, $Ce^{4+}$, $V^{3+}$, $Co^{2+}$, $Ru^{3+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Besonders bevorzugte Mittel 0,0001 bis 2,5 Gew.-%, vorzugsweise 0,001 bis 1 Gew.-% mindestens einer Verbindung aus der Gruppe Kupferchlorid ($CuCl_2$), Kupfersulfat ($CuSO_4$), Eisen(II)sulfat, Mangan(II)sulfat, Mangan(II)chlorid, Kobalt(II)chlorid, Cersulfat, Cerchlorid, Vanadiumsulfat, Mangandioxid ($MnO_2$) enthalten.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab.

Im Rahmen der vorliegenden Erfindung können alle Komplexbildner des Standes der Technik eingesetzt werden. Diese können unterschiedlichen chemischen Gruppen angehören. Vorzugsweise werden einzeln oder im Gemisch miteinander eingesetzt:

Polycarbonsäuren, bei denen die Summe der Carboxyl- und gegebenenfalls Hydroxylgruppen mindestens 5 beträgt wie Gluconsäure,

stickstoffhaltige Mono- oder Polycarbonsäuren wie Ethylendiamintetraessigsäure (EDTA), N-Hydroxyethylethylendiamintriessigsäure, Diethylentriaminpentaessigsäure, Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)-glycin, N-(1,2-Dicarboxy-2-hydroxyethyl)asparaginsäure oder Nitrilotriessigsäure (NTA),

geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppenhaltige Derivate hiervon,

Aminophosphonsäuren wie Ethylendiamintetra(methylenphosphonsäure), Diethylentriaminpenta(methylenphosphonsäure) oder Nitrilotri(methylenphosphonsäure),

Phosphonopolycarbonsäuren wie 2-Phosphonobutan-1,2,4-tricarbonsäure sowie Cyclodextrine.

Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Wie bereits erwähnt, können die erfindungsgemäßen Mittel nicht nur als reine Aufhellmittel, d.h. als sogenannte Blondiermittel, sondern auch als Färbe- und Aufhellmittel bereitgestellt werden, die gleichzeitig mit der Aufhellung auch eine Färbung der Keratinfasern bewirken.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen

Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z. B. Haare, aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

In einer Ausführungsform zur Farbveränderung ist der Gegenstand der vorliegenden Erfindung mit mindestens einer farbverändernden Komponente kombinierbar. Die farbverändernden Komponenten im Sinne der vorliegenden Erfindung werden bevorzugt ausgewählt

(1) aus mindestens einem Oxidationsfarbstoffvorprodukt
und/oder
(2) aus mindestens einem direktziehenden Farbstoff.

Zu diesem Zweck enthalten solche erfindungsgemäßen Mittel mindestens ein Farbstoffvorprodukt, vorzugsweise ein Oxidationsfarbstoffvorprodukt und/oder mindestens einen direktziehenden Farbstoff. Unter diesen sind insbesondere die sogenannten Oxidationsfärbemittel bevorzugt.

Die erfindungsgemäßen Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten.

Erfindungsgemäß bevorzugte Mittel zum Färben und/oder Aufhellen keratinischer Fasern sind demnach dadurch gekennzeichnet, dass sie mindestens ein Oxidationsfarbstoffvorprodukt vom Entwicklertyp und/oder Kupplertyp enthalten.

Bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol und 4-Amino-2-(diethyl-aminomethyl)-phenol, 4,5-Diamino-1-(2-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)-propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)-ethanol, 2-({3-[(2-Hydroxyethyl)amino]-4,5-dimethylphenyl}amino)ethanol, 2-[3-Morpholin-4-yl-phenyl)amino]ethanol, 3-Amino-4-(2-methoxyethoxy)-5-methylphenylamin, 1-Amino-3-bis-(2-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-di-methylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Entwickler- und Kupplerkomponenten werden bevorzugt jeweils in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss

einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden. Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen. Bevorzugte kationische direktziehende Farbstoffe sind dabei kationische Triphenylmethanfarbstoffe, wie beispielsweise Basic Blue 7, Basic Blue 26, Basic Violet 2 und Basic Violet 14; aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe. Die kationischen direktziehenden Farbstoffe, die unter dem Warenzeichen Arianor® vertrieben werden, sind erfindungsgemäß ebenfalls ganz besonders bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Ferner können die erfindungsgemäßen Mittel weitere Wirk-, Hilfs- und Zusatzstoffe enthalten, wie nichtionische Polymere, kationische Polymere, zwitterionische und amphotere Polymere, anionische Polymere, Verdickungsmittel, Strukturanten, haarkonditionierende Verbindungen, Proteinhydrolysate, Parfümöle, Dimethylisosorbid und Cyclodextrine, faserstrukturverbessernde Wirkstoffe, Entschäumer wie Silikone, Farbstoffe zum Anfärben des Mittels, Antischuppenwirkstoffe wie Piroctone Olamine, Zink Omadine und Climbazol, Lichtschutzmittel, insbesondere Derivate von Benzophenon, Zimtsäure und Triazin, Wirkstoffe wie Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, $B_3$, $B_5$, $B_6$, C, E, F und H, Pflanzenextrakte, Cholesterin, Konsistenzgeber wie Zuckerester, Polyolester oder Polyolalkylether, Fette und Wachse wie Walrat, Bienenwachs, Montanwachs und Paraffine, Fettsäurealkanolamide, Quell- und Penetrationsstoffe, Trübungsmittel wie Latex, Styrol/PVP- und Styrol/ Acrylamid-Copolymere, Perlglanzmittel, Pigmente, Stabilisierungsmittel für Wasserstoffperoxid und andere Oxidationsmittel, Treibmittel wie Propan-Butan-Gemische, $N_2O$, Dimethylether, $CO_2$ und Luft, Antioxidantien.

Die Auswahl dieser weiteren Stoffe wird der Fachmann gemäß der gewünschten Eigenschaften der Mittel treffen.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Die erfindungsgemäßen Mittel enthalten die Wirkstoffe in einem kosmetischen Träger. Dieser kosmetische Träger ist bevorzugt wässrig, alkoholisch oder wässrig-alkoholisch. Zum Zwecke der Haarbleiche sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch Tabletten-förmige Formulierung bereitzustellen, was für Aufhellmittel bevorzugt ist. Diese wird dann vor der Anwendung in einem Lösemittel, wie Wasser oder mit organischen Lösemitteln bzw. mit Gemischen aus Wasser und organischen Lösemitteln unter Erhalt der Anwendungsmischung vermengt.

Ein wässriger Träger enthält im Sinne der Erfindung mindestens 40 Gew.-%, insbesondere mindestens 50 Gew.-% Wasser.

Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.

Bevorzugte erfindungsgemäße Mittel sind dadurch gekennzeichnet, dass sie zusätzlich ein nichtwässriges Lösungsmittel enthalten, wobei besonders bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Ein zweiter Gegenstand der vorliegenden Erfindung ist ein Verfahren zum Aufhellen von Keratinfasern, insbesondere menschlichen Haaren, dadurch gekennzeichnet, dass ein Mittel des ersten Erfindungsgegenstandes auf die keratinhaltigen Fasern aufgebracht, 5 bis 60 Minuten auf der Faser belassen und anschließend wieder ausgespült oder mit einem Shampoo ausgewaschen wird. Insbesondere liegt die Temperatur während der Einwirkzeit von 5 bis 60 Minuten zwischen 10 °C und 40 °C, insbesondere zwischen 20 °C und 38 °C.

Im Rahmen eines solchen Verfahren kann es bevorzugt sein, das Verfahren dadurch zu kennzeichnen, dass gewünschtenfalls ein Vorbehandlungsmittel M1 auf die Faser aufgebracht wird, dann
ein Mittel M2 auf der Faser zur Anwendung kommt, wobei gewünschtenfalls dem Mittel M2 vor der Anwendung ein weiteres Mittel M3 zugegeben wird,
dieses Mittel M2 nach einer Zeit von 5 bis 60 Minuten von der Faser abgespült wird
und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 oder die Mischung der Mittel M2 und M3 ein erfindungsgemäßes Mittel des ersten Erfindungsgegenstandes ist.

Die erfindungsgemäßen Mittel können demnach als Einkomponentenmittel (Färbe- und/oder Aufhellmittel M2), als Zweikomponenten Mittel (M2 + M3) formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Färbe- und/oder Aufhellungsverfahren, bei dem die Verbindungen der allgemeinen Struktur (I) und des Co-Bleichaktivators zunächst getrennt von Wasserstoffperoxid vorliegen, ist dabei bevorzugt. Ein weiterer Gegenstand der vorliegenden Erfindung ist daher ein Verfahren zum Aufhellen und gegebenenfalls Färben von menschlichen Haaren, bei dem eine Zusammensetzung auf wässriger Grundlage, enthaltend Wasserstoffperoxid, mit einer Zusammensetzung, enthaltend mindestens eine Verbindung der allgemeinen Struktur (I) und mindestens ein toxikologisch unbedenklicher Co-Bleichaktivator (*vide supra*) zu einem Mittel der ersten Erfindungsgegenstandes vermischt, und dieses auf das Haar aufgebracht wird.

Eine weitere Ausführungsform des erfindungsgemäßen Verfahrens zum Aufhellen und gegebenenfalls Färben von menschlichen Haaren liegt vor, wenn eine Zusammensetzung auf wässriger Grundlage, enthaltend Wasserstoffperoxid, mit einem weiteren Mittel enthaltend vorzugsweise mindestens einen Alkalitätsgeber und/oder direktziehenden Haarfarbstoff und/oder mindestens ein Oxidationsfarbstoffvorprodukt, und einem Mittel enthaltend eine Verbindung der allgemeinen Struktur (I) (*vide supra*) und zusätzlich einen Co-Bleichaktivator (*vide supra*) zu einer homogenen Zusammensetzung vermischt, und diese auf das Haar aufgebracht wird.

Ein dritter Gegenstand der Erfindung ist die Verwendung der Mittel des ersten Erfindungsgegenstandes zum Aufhellen von keratinhaltigen Fasern, insbesondere menschlichen Haaren.

Bezüglich weiterer bevorzugter Ausführungsformen der erfindungsgemäßen Verfahren bzw. der erfindungsgemäßen Verwendung gilt mutatis mutandis das zu den erfindungsgemäßen Mitteln Gesagte.

Beispiele

1.0 Synthese von 4-Acetyl-1-methylpryidinium-p-toluolsulfonat

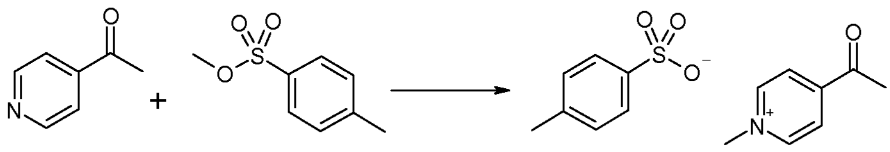

Es wurden 30,0 g (0,25 mol) 4-Acetylpyridin und 55,8 g (0,30 mol) p-Toluolsulfonsäure-methylester in 500 ml Ethanol für 5 Stunden unter Rückfluss erhitzt. Das Lösungsmittel wurde im Vakuum am Rotationsverdampfer abgezogen und der Rückstand mit Ether digeriert. Nach Abtrennen der Etherphase kristallisierte das Produkt nach und nach aus. Das Produkt wurde im Vakuum getrocknet. Ausbeute: 59,9 g (82,5 %); $^{1}$H-NMR (400 MHz, DMSO-d6): $\delta$ [ppm] = 2,26 (s, 3H); 2,72 (s, 3H); 3,39 (s, 3H); 7,11 (d, 2H); 7,49 (d, 2H); 8,42 (d, 2H); 9,20 (d, 2H); $^{13}$C-NMR (400 MHz, DMSO-d6): $\delta$ [ppm] = 20,8; 26,4; 48,1; 124,8; 125,3; 127,7; 138,9; 145,2; 146,5; 148,3; 195,8.

2. Beispiele zur Blondierung

2.1. Blondierungen mit Wasserstoffperoxid und Co-Bleichaktivator

2.1.1. Herstellung einer Blondiercreme

Aus den aufgelisteten Bestandteilen wurden Blondiercremes wie folgt hergestellt:

| Rohstoff | Gew.-% | | | | |
|---|---|---|---|---|---|
| | **V1** | **V2** | **E1** | **E2** | **E3** |
| Hydrenol D | 6,9 | 6,9 | 6,9 | 6,9 | 6,9 |
| Lorol techn. | 2,5 | 2,5 | 2,5 | 2,5 | 2,5 |
| Eumulgin B1 | 0,6 | 0,6 | 0,6 | 0,6 | 0,6 |
| Eumulgin B2 | 0,6 | 0,6 | 0,6 | 0,6 | 0,6 |
| Akypo Soft 45 NV | 10,0 | 10,0 | 10,0 | 10,0 | 10,0 |
| Plantacare 1200 UP | 2,0 | 2,0 | 2,0 | 2,0 | 2,0 |
| Texapon K 14 S 70 C | 2,8 | 2,8 | 2,8 | 2,8 | 2,8 |
| Ammoniumsulfat | 1,0 | 1,0 | 1,0 | 1,0 | 1,0 |
| Ascorbinsäure | 0,1 | 0,1 | 0,1 | 0,1 | 0,1 |
| Natriumsilikat 40 / 42 | 0,5 | 0,5 | 0,5 | 0,5 | 0,5 |
| Turpinal SL | 0,2 | 0,2 | 0,2 | 0,2 | 0,2 |
| KOH | 0,8 | 0,8 | 0,8 | 0,8 | 0,8 |
| Ammoniak, 25 Gew.-% wässrig | 7,1 | 7,1 | 7,1 | 7,1 | 7,1 |
| Glycin | **-** | **-** | **2,0** | **-** | **-** |
| Benzylalkohol | **-** | **-** | **-** | **2,0** | **-** |
| Disponil FES 77 (Aktivsubstanz) | **-** | **-** | **-** | **-** | **2,0** |
| 4-Acetyl-1-methylpyridinium-p-toluolsulfonat (gemäß Beispiel 1.1) | **-** | **2,0** | **2,0** | **2,0** | **2,0** |
| Wasser | ad 100 | ad 100 | ad 100 | ad 100 | ad 100 |

| | |
|---|---|
| Hydrenol® D | $C_{16}$-$C_{18}$-Fettalkohol (INCI-Bezeichnung: Cetearyl alcohol) (Cognis) |
| Lorol® tech. | $C_{12}$-$C_{18}$-Fettalkohol (INCI-Bezeichnung: Coconut alcohol) (Cognis) |
| Eumulgin® B1 | $C_{16}$-$C_{18}$-Fettalkohol, ethoxyliert (12 EO) (INCI-Bezeichnug: Ceteareth-12) (Cognis) |
| Eumulgin® B2 | $C_{16}$-$C_{18}$-Fettalkohol, ethoxyliert (20 EO) (INCI-Bezeichnug: Ceteareth-20) (Cognis) |
| Akypo® Soft 45 NV | $C_{12}$-$C_{14}$-Fettalkoholetheressigsäure, Natriumsalz (4,5 EO) (INCI-Bezeichnung: Sodium Laureth-5 carboxylate) (KAO Chemicals) |
| Plantacare® 1200 UP | $C_{12}$-$C_{16}$-Fettalkoholglucoside (INCI-Bezeichnung: Lauryl Glucoside) (Cognis) |
| Texapon® K 14 S 70 C | Myristylethersulfat, Natriumsalz (ca. 70% Aktivsubstanz; INCI-Bezeichnung: Sodium Myreth Sulfate) (Cognis) |
| Turpinal® SL | 1-Hydroxyethan-1,1-diphosphonsäure (ca. 60% Aktivsubstanzgehalt; INCI-Bezeichnung: Etidronic Acid, Aqua (Water)) (Solutia) |
| Natriumsilikat 40/42 | Natronwasserglas |
| Disponil® FES 77 | $C_{12}$-$C_{18}$-Fettalkoholethersulfat, Natriumsalz (30 EO) (ca. 31-33% Aktivsubstanz in Wasser; INCI-Bezeichnung: Sodium Coceth-30 Sulfate) (Cognis) |

Hydrenol D, Lorol, Eumulgin B1, Eumulgin B2, Akypo Soft 45 NV, Plantacare 1200 UP und Texapon K 14 S 70 C wurden zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

Bei den Rezepturen V1 und V2 handelt es sich um nicht erfindungsgemäße Vergleichsrezepturen ohne Co-Bleichaktivator, die Rezepturen E1 bis E3 sind erfindungsgemäße Beispiel mit dem Bleichaktivator 4-Acetyl-1-methylpyridinium-p-toluolsulfonat und Co-Bleichaktivatoren.

2.1.2. Vermischen mit der Entwicklerdispersion

Jede Blondiercreme wurde im Verhältnis 1:1 mit einer wie folgt zusammengesetzten Entwicklerdispersion ausgemischt. Der pH-Wert der fertigen Anwendungsmischung lag zwischen 9 und 10,2.

| **Rohstoff** | **Gew.-%** |
|---|---|
| Ammoniak 25 % | 0,62 |
| Dipicolinsäure | 0,10 |
| Dinatriumpyrophosphat | 0,03 |
| Turpinal SL | 1,50 |
| Texapon NSO | 2,00 |
| Dow Corning DB 110 A | 0,07 |
| Aculyn 33A (Acrylpolymer) | 12,00 |
| Wasserstoffperoxid 50 % | 22,40 |
| Wasser | ad 100 |

| | |
|---|---|
| Texapon® NSO | Laurylethersulfat, Natriumsalz (ca. 27,5% Aktivsubstanz; INCI-Bezeichnung: Sodium Laureth Sulfate) (Cognis) |
| Aculyn® 33 | Acrylpolymer (ca. 28% Festkörper in Wasser; INCI-Bezeichnung: Acrylates Copolymer) (Rohm & Haas) |
| Dow Corning® DB 110 A | nicht ionische Silikonemulsion (INCI-Bezeichnung: Dimethicon) (Dow Corning) |

Für den Blondierprozess wurde auf Strähnen dunkelblonden, hellbraunen und dunkelbraunen Haares (Codes: Kerling 7/0, Fischbach & Miller 6923 und Kerling 2/0) von ca. 0,7 g Gewicht die 4-fache Menge der fertigen Anwendungsmischung appliziert. Nachdem die Strähnen für 30 min bei 32 °C blondiert wurden, wurden sie mit einem handelsüblichen Shampoo gewaschen und mit einem Föhn getrocknet.

2.1.3. Auswertung der Aufhellleistung

Jede Haarsträhne wurde vor und nach dem Bleichvorgang farbmetrisch vermessen. Als Maß für die Aufhellleistung der jeweiligen Rezeptur wurde der dL-Wert gemäß folgender Formel herangezogen:

$$dL = L_{nachher} - L_{vorher}$$

mit $L_{nachher}$ = Helligkeit der Strähne nach dem Bleichen; $L_{vorher}$ = Helligkeit der Strähne vor dem Bleichen

Für jede Rezeptur und jeden Haartyp erfolgte eine Zwölffachbestimmung, aus den Einzelwerten wurde jeweils der Mittelwert gebildet. Je größer der dL-Wert ist, desto besser ist die Aufhellleistung der jeweiligen Rezeptur.

Aufhellleistung bei dunkelblonden Strähnen (Kerling 7/0)

| dL (V1) | dL (V2) | dL (E1) | dL (E2) | dL (E3) |
|---|---|---|---|---|
| **10,8** | **12,6** | **12,8** | **13,6** | **13,2** |

Aufhellleistung bei hellbraunen Strähnen (Fischbach & Miller 6923)

| dL (V1) | dL (V2) | dL (E1) | dL (E2) | dL (E3) |
|---|---|---|---|---|
| **12,1** | **12,8** | **13,6** | **14,1** | **13,8** |

Aufhellleistung bei dunkelbraunen Strähnen (Kerling 2/0)

| dL (V1) | dL (V2) | dL (E1) | dL (E2) | dL (E3) |
|---|---|---|---|---|
| **5,3** | **6,3** | **8,0** | **6,8** | **7,2** |

2.1.4 Deutung der Ergebnisse

Eine Abschätzung der Bleichwirkungen der unterschiedlichen Rezepturen lässt sich durch den Vergleich der dL-Werte treffen. Es ist eindeutig ersichtlich, dass mit der erfindungsgemäßen Kombination aus Wasserstoffperoxid, dem spezifischen Co-Bleichaktivator und dem kationischen Acylpyridiniumderivat signifikant größere dL Werte - und damit eine bessere Aufhellung - erzielt werden konnten, als es durch den Einsatz von Wasserstoffperoxid alleine oder in Kombination mit dem Acylpyridiniumderivat möglich war. Durch den Einsatz dieser speziellen Kombination aus drei Komponenten konnte daher gegenüber dem existierenden Stand der Technik eine wesentliche Verbesserung erzielt werden.

**Patentansprüche**

1. Mittel zum Aufhellen von keratinischen Fasern, **dadurch gekennzeichnet, dass** es in einem kosmetischen Träger

(i) mindestens ein kationisches Acylpyridiniumderivat der Formel (I)

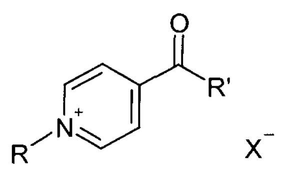

(I)

worin

R für eine $C_1$-$C_6$-Alkylgruppe, eine $C_2$-$C_6$-Alkenylgruppe, oder eine $C_2$-$C_6$-Hydroxyalkyl-gruppe, steht,
R' für eine $C_1$-$C_4$-Alkylgruppe, eine $C_2$-$C_6$-Hydroxyalkylgruppe oder eine $C_1$-$C_6$-Alkoxy-$C_2$-$C_6$-alkylgruppe,
$X^-$ für ein physiologisch verträgliches Anion steht,

(ii) mindestens einen toxikologisch unbedenklichen Co-Bleichaktivator und/oder dessen physiologisch verträgliches Salz, und
(iii) Wasserstoffperoxid enthält.

**2.** Mittel nach Anspruch 1, **dadurch gekennzeichnet, dass** mindestens eine Verbindung der Formel (I) enthalten ist, die ausgewählt wird aus mindestens einer Verbindung der Gruppe, die gebildet wird aus

4-Acetyl-1-methylpyridinium-p-toluolsulfonat
4-Acetyl-1-methylpyridiniumbenzolsulfonat
4-Acetyl-1-methylpyridiniumbromid
4-Acetyl-1-methylpyridiniumhydrogensulfat
4-Acetyl-1-allylpyridinium-p-toluolsulfonat
4-Acetyl-1-allylpyridiniumbenzolsulfonat
4-Acetyl-1-allylpyridiniumbromid
4-Acetyl-1-allylpyridiniumhydrogensulfat
4-Acetyl-1-(2-hydroxyethyl)-pyridinium-p-toluolsulfonat
4-Acetyl-1-(2-hydroxyethyl)-pyridiniumbenzolsulfonat
4-Acetyl-1-(2-hydroxyethyl)-pyridiniumbromid
4-Acetyl-1-(2-hydroxyethyl)-pyridiniumhydrogensulfat

**3.** Mittel nach einem der Ansprüche 1 bis 2, **dadurch gekennzeichnet, dass** die Acylpyridiniumderivate der Formel (I) in einer Menge von 0,01 bis 25 Gew.-%, insbesondere von 0,1 bis 10 Gew.-%, jeweils bezogen auf das Gesamtgewicht des anwendungsbereiten Mittels, enthalten sind.

**4.** Mittel nach einem der Ansprüche 1 bis 3, **dadurch gekennzeichnet, dass** es als Co-Bleichaktivator und/oder dessen physiologisch verträgliches Salz mindestens einen aliphatischen und/oder carbocyclischen Co-Bleichaktivator enthält.

**5.** Mittel nach einem der Ansprüche 1 bis 4, **dadurch gekennzeichnet, dass** der Co-Bleichaktivator und/oder dessen physiologisch verträgliches Salz mindestens eine funktionelle Gruppe, ausgewählt aus einer Hydroxygruppe, Carbonsäure, Schwefelsäuremonoester, oder Phosphorsäuremonoester, enthält.

**6.** Mittel nach einem der Ansprüche 1 bis 5, **dadurch gekennzeichnet, dass** es als Co-Bleichaktivator mindestens einen Co-Bleichaktivator gemäß Formel (II) und/oder dessen physiologisch verträgliches Salz enthält,

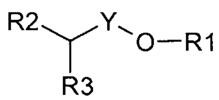
(II)

worin

Y für eine Carbonyl-Gruppe, eine direkte Bindung oder Methylen-Gruppe steht,
R1 für Wasserstoff, eine $C_1$-$C_4$-Alkylgruppe, ein physiologisch verträgliches Kation oder eine $SO_3^-$- oder eine $PO_3^{2-}$- Gruppe steht,
R2 für eine Amino-, eine Methylamino-, eine Dimethylamino-, eine Trimethylammonio-Gruppe, Phenyl, Benzyl, Phenoxymethyl, 1-Naphthyl, 2-Naphthyl, 2-, 3-, 4-Toluolyl, oder eine R4-O-$(CH_2CH_2O)_n$-Gruppe steht, worin R4 für eine $C_6$-$C_{20}$-Alkylgruppe und n für eine Zahl von größer als 15 steht,
R3 für Wasserstoff oder eine gegebenenfalls verzweigte $C_1$-$C_6$-Alkylgruppe steht, mit der Maßgabe, dass,

wenn Y für eine Carbonyl-Gruppe steht,
R1 für Wasserstoff, eine $C_1$-$C_4$-Alkylgruppe oder ein physiologisch verträgliches Kation steht,
R2 für eine Amino-, eine Methylamino-, eine Dimethylamino- oder eine Trimethylammonio-Gruppe steht, und
R3 für Wasserstoff oder eine gegebenenfalls verzweigte $C_1$-$C_6$-Alkylgruppe steht, dass,
wenn Y für eine direkte Bindung steht,
R1 für Wasserstoff und
R2 für Phenyl, Benzyl, Phenoxymethyl, 1-Naphthyl, 2-Naphthyl, 2-, 3- oder 4-Toluolyl steht, und
R3 für Wasserstoff oder eine gegebenenfalls verzweigte $C_1$-$C_6$-Alkylgruppe steht, und dass,

wenn Y für eine Methylen-Gruppe steht,
R1 für eine $SO_3^-$- oder eine $PO_3^{2-}$-Gruppe steht,
R2 für eine R4-$O(CH_2CH_2O)_n$-Gruppe steht, worin R4 für eine $C_6$-$C_{20}$-Alkylgruppe und n für eine Zahl von größer als 15 steht, und
R3 für Wasserstoff steht.

7. Mittel nach einem der Ansprüche 1 bis 6, **dadurch gekennzeichnet, dass** als Co-Bleichaktivator Glycin und/oder dessen physiologisch verträgliches Salz enthalten ist.

8. Mittel nach einem der Ansprüche 1 bis 6, **dadurch gekennzeichnet, dass** es als Co-Bleichaktivator Benzylalkohol enthält.

9. Mittel nach einem der Ansprüche 1 bis 6, **dadurch gekennzeichnet, dass** es als Co-Bleichaktivator ein physiologisch verträgliches Salz von R4-$O(CH_2CH_2O)_m$-$SO_3^-$ enthält, worin R4 für eine $C_6$-$C_{20}$-Alkylgruppe und m für eine Zahl von größer als 15 steht.

10. Mittel nach einem der Ansprüche1 bis 9, **dadurch gekennzeichnet, dass** der Co-Bleichaktivator und/oder dessen physiologisch verträgliches Salz in einer Menge von 0,01 bis 10 Gew.-%, insbesondere von 0,1 bis 5 Gew.-%, bezogen auf das Gesamtgewicht des anwendungsbereiten Mittels, vorliegt.

11. Mittel nach einem der Ansprüche 1 bis 10, **dadurch gekennzeichnet, dass** es einen pH-Wert von 7 bis 11 besitzt.

12. Mittel nach einem der Ansprüche 1 bis 11, **dadurch gekennzeichnet, dass** zusätzlich mindestens ein anorganisches Persulfat- oder Peroxodisulfat-Salz, insbesondere Ammoniumperoxodisulfat, Kaliumperoxodisulfat und/oder Natriumperoxodisulfat, enthalten ist.

13. Verfahren zum Aufhellen von keratinischen Fasern, **dadurch gekennzeichnet, dass** ein Mittel nach einem der Ansprüche 1 bis 12 auf die keratinhaltigen Fasern aufgebracht, 5 bis 60 Minuten auf der Faser belassen und anschließend wieder ausgespült oder mit einem Shampoo ausgewaschen wird.

14. Kosmetische Verwendung eines Mittels nach einem der Ansprüche 1 bis 12 zum Aufhellen von keratinhaltigen Fasern, insbesondere menschlichen Haaren.

## Claims

1. An agent for lightening keratin fibers, **characterized in that** said agent contains, in a cosmetic carrier,

(i) at least one cationic acylpyridinium derivative of formula (I)

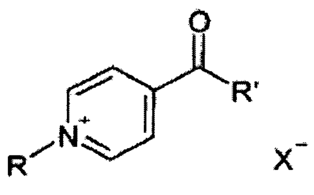

(I)

in which

R represents a $C_1$-$C_6$ alkyl group, a $C_2$-$C_6$ alkenyl group, or a $C_2$-$C_6$ hydroxyalkyl group
R' represents a $C_1$-$C_4$ alkyl group, a $C_2$-$C_6$ hydroxyalkyl group or a $C_1$-$C_6$-alkoxy-$C_2$-$C_6$-alkyl group,
X- represents a physiologically acceptable anion,

(ii) at least one toxicologically harmless co-bleach activator and/or a physiologically acceptable salt thereof, and
(iii) hydrogen peroxide.

2. The agent according to claim 1, **characterized in that** said agent contains at least one compound of formula (I) which is selected from at least one compound from the group formed by:

4-acetyl-1-methylpyridinium-p-toluenesulfonate
4-acetyl-1-methylpyridinium benzenesulfonate
4-acetyl-1-methylpyridinium bromide
4-acetyl-1-methylpyridinium hydrogen sulfate
4-acetyl-1-allylpyridinium-p-toluenesulfonate
4-acetyl-1-allylpyridinium benzenesulfonate
4-acetyl-1-allylpyridinium bromide
4-acetyl-1-allylpyridinium hydrogen sulfate
4-acetyl-1-(2-hydroxyethyl)-pyridinium-p-toluenesulfonate
4-acetyl-1-(2-hydroxyethyl)-pyridinium benzenesulfonate
4-acetyl-1-(2-hydroxyethyl)-pyridinium bromide
4-acetyl-1-(2-hydroxyethyl)-pyridinium hydrogen sulfate

3. The agent according to one of claims 1 to 2, **characterized in that** said agent contains the acylpyridinium derivatives of formula (I) in an amount of from 0.01 to 25 wt.%, in particular from 0.1 to 10 wt.%, in each case based on the total weight of the ready-to-use agent.

4. The agent according to one of claims 1 to 3, **characterized in that** said agent contains as the co-bleach activator and/or a physiologically acceptable salt thereof at least one aliphatic and/or carbocyclic co-bleach activator.

5. The agent according to one of claims 1 to 4, **characterized in that** the co-bleach activator and/or a physiologically acceptable salt thereof contains at least one functional group selected from a hydroxy group, carboxylic acid, sulfuric acid monoester or phosphoric acid monoester.

6. The agent according to one of claims 1 to 5, **characterized in that** said agent contains as the co-bleach activator at least one co-bleach activator according to formula (II) and/or a physiologically acceptable salt thereof,

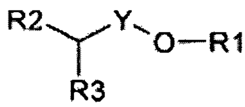

(II)

in which

Y represents a carbonyl group, a direct bond, or a methylene group,
R1 represents hydrogen, a $C_1$-$C_4$ alkyl group, a physiologically acceptable cation or a $SO_3^-$ or $PO_3^{2-}$ group,
R2 represents an amino group, a methylamino group, a dimethylamino group, a trimethylammonio group, phenyl, benzyl, phenoxymethyl, 1-naphthyl, 2-naphthyl, 2-, 3-, 4-toluolyl, or an R4-O-$(CH_2CH_2O)_n$ group, in which R4 represents a $C_6$-$C_{20}$ alkyl group and n represents an integer greater than 15,
R3 represents hydrogen or an optionally branched $C_1$-$C_6$ alkyl group, with the proviso that
when Y represents a carbonyl group,
R1 represents hydrogen, a $C_1$-$C_4$ alkyl group or a physiologically acceptable cation, R2 represents an amino group, a methylamino group, a dimethylamino group or a trimethylammonio group, and
R3 represents hydrogen or an optionally branched $C_1$-$C_6$ alkyl group,
that
when Y represents a direct bond,
R1 represents hydrogen and
R2 represents phenyl, benzyl, phenoxymethyl, 1-naphthyl, 2-naphthyl, 2-, 3-, or 4-toluolyl, and
R3 represents hydrogen or an optionally branched $C_1$-$C_6$ alkyl group,
and that
when Y represents a methylene group,
R1 represents a $SO_3^-$ or $PO_3^{2-}$ group,
R2 represents a R4-O$(CH_2CH_2O)_n$ group, in which R4 represents a $C_6$-$C_{20}$ alkyl group and n represents an integer greater than 15, and
R3 represents hydrogen.

7. The agent according to one of claims 1 to 6, **characterized in that** said agent contains as the co-bleach activator glycine and/or a physiologically acceptable salt thereof.

8. The agent according to one of claims 1 to 6, **characterized in that** said agent contains benzyl alcohol as the co-bleach activator.

9. The agent according to one of claims 1 to 6, **characterized in that** said agent contains as the co-bleach activator a physiologically acceptable salt of $R4\text{-}O(CH_2CH_2O)_m\text{-}SO_3^-$ in which R4 represents a $C_6$-$C_{20}$ alkyl group and m represents an integer greater than 15.

10. The agent according to one of claims 1 to 9, **characterized in that** the co-bleach activator and/or a physiologically acceptable salt thereof is present in an amount of from 0.01 to 10 wt.%, in particular from 0.1 to 5 wt.%, based on the total weight of the ready-to-use agent.

11. The agent according to one of claims 1 to 10, **characterized in that** said agent has a pH of from 7 to 11.

12. The agent according to one of claims 1 to 11, **characterized in that** said agent additionally contains at least one inorganic persulfate salt or peroxodisulfate salt, in particular ammonium peroxodisulfate, potassium peroxodisulfate and/or sodium peroxodisulfate.

13. A method for lightening keratin fibers, **characterized in that** an agent according to one of claims 1 to 12 is applied to the keratin-containing fibers, is left on the fibers for from 5 to 60 minutes and is then rinsed off again or washed out with a shampoo.

14. The cosmetic use of an agent according to one of claims 1 to 12 for lightening keratin-containing fibers, in particular human hair.

**Revendications**

1. Agent d'éclaircissement de fibres de kératine, **caractérisé en ce qu'**il contient, dans un support cosmétique,

(i) au moins un dérivé cationique du pyridinium d'acyle de la formule (I)

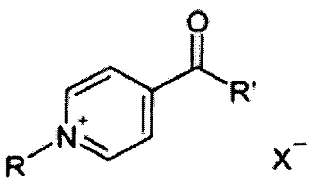

(I)

où

R représente un groupe alkyle en $C_1$ à $C_6$, un groupe alcényle en $C_2$ à $C_6$ ou un groupe hydroxyalkyle en $C_2$ à $C_6$,
R' est un groupe alkyle en $C_1$ à $C_4$, un groupe hydroxyalkyle en $C_2$ à $C_6$ ou un groupe alkyle en $C_1$ à $C_6$-alcoxy en $C_2$ à $C_6$,
X- représente un anion physiologiquement acceptable,

(ii) au moins un co-activateur de blanchiment sans danger du point de vue toxicologique et/ou un sel physiologiquement acceptable de celui-ci,
et
(iii) du peroxyde d'hydrogène.

2. Agent selon la revendication 1, **caractérisé en ce qu'**il contient au moins un composé de la formule (I) qui est choisi parmi au moins un composé du groupe formé par

le p-toluène-sulfonate de 4-acétyl-1-méthylpyridinium
le benzène-sulfonate de 4-acétyle-1-méthylpyridinium
le bromure de 4-acétyl-1-méthylpyridinium
l'hydrogénosulfate de 4-acétyl-1-méthylpyridinium
le p-toluène-sulfonate de 4-acétyl-1-allylpyridinium
le benzène-sulfonate de 4-acétyl-1-allylpyridinium
le bromure de 4-acétyl-1-allylpyridinium
l'hydrogénosulfate de 4-acétyl-1-allylpyridinium
le p-toluène-sulfonate de 4-acétyl-1-(2-hydroxyéthyl)-pyridinium
le benzène-sulfonate de 4-acétyl-1-(2-hydroxyéthyl)-pyridinium
le bromure de 4-acétyl-1-(2-hydroxyéthyl)-pyridinium
l'hydrogénosulfate de 4-acétyl-1-(2-hydroxyéthyl)-pyridinium

**3.** Agent selon l'une des revendications 1 à 2, **caractérisé en ce que** les dérivés de pyridinium d'acyle de la formule (I) sont contenu dans une quantité de 0,01 à 25% en poids, en particulier de 0,1 à 10% en poids, à chaque fois par rapport au poids total de l'agent prêt à l'emploi.

**4.** Agent selon l'une des revendications 1 à 3, **caractérisé en ce qu'**il contient comme co-activateur de blanchiment et/ou un sel physiologiquement acceptable de celui-ci au moins un co-activateur de blanchiment aliphatique et/ou carbocyclique.

**5.** Agent selon l'une des revendications 1 à 4, **caractérisé en ce que** le co-activateur de blanchiment et/ou un sel physiologiquement acceptable de celui-ci contient au moins un groupe fonctionnel choisi parmi un groupe hydroxy, un acide carboxylique, un monoester d'acide sulfurique ou un monoester d'acide phosphorique.

**6.** Agent selon l'une des revendications 1 à 5, **caractérisé en ce qu'**il contient comme co-activateur de blanchiment au moins un co-activateur de blanchiment selon la formule (II) et/ou un sel physiologiquement acceptable de celui-ci,

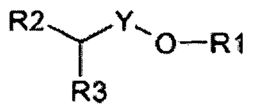

**(II)**

où

Y représente un groupe carbonyle, une liaison directe ou un groupe méthylène,
R1 représente un hydrogène, un groupe alkyle en $C_1$ à $C_4$, un cation physiologiquement acceptable ou un groupe $SO_3$- ou $PO_3^{2-}$-,
R2 représente un groupe amino-, méthylamino-, diméthylamino-, triméthylammonio-, phényle, benzyle, phénoxyméthyle, 1-naphtyle, 2-naphtyle, 2-, 3-, 4-toluoyle, ou un groupe R4-O-$CH_2CH_2O)_n$, où R4 représente un groupe alkyle en $C_6$ à $C_{20}$ et n est un nombre supérieur à 15,
R3 représente un hydrogène ou un groupe alkyle en $C_1$ à $C_6$ éventuellement ramifié, avec la condition que lorsque Y représente un groupe carbonyle,
R1 représente un hydrogène, un groupe alkyle en $C_1$ à $C_4$ ou un cation physiologiquement acceptable,
R2 représente un groupe amino, méthylamino, diméthylamino ou triméthylammonio, et
R3 représente un hydrogène ou un groupe alkyle en $C_1$ à $C_6$ éventuellement ramifié, lorsque Y représente une liaison directe,
R1 représente un hydrogène et
R2 représente un groupe phényle, benzyle, phénoxyméthyle, 1-naphtyle, 2-naphtyle, 2-, 3- ou 4-toluoyle, et
R3 représente un hydrogène ou un groupe alkyle en $C_1$ à $C_6$ éventuellement ramifié,
et
lorsque Y représente un groupe méthylène,
R1 représente un groupe $SO_3$- ou $PO_3^{2-}$,
R2 représente un groupe R4-O $CH_2CH_2O)n$, où R4 représente un groupe alkyle en $C_6$ à $C_{20}$ et n est un nombre supérieur à 15, et
R3 représente un hydrogène.

**7.** Agent selon l'une des revendications 1 à 6, **caractérisé en ce qu'**il contient de la glycine et/ou un sel physiologiquement acceptable de celle-ci comme co-activateur de blanchiment.

8. Agent selon l'une des revendications 1 à 6, **caractérisé en ce qu'**il contient de l'alcool benzylique comme co-activateur de blanchiment.

9. Agent selon l'une des revendications 1 à 6, **caractérisé en ce qu'**il contient comme co-activateur de blanchiment un sel physiologiquement acceptable de $R4-O(CH_2CH_2O)_m-SO_3-$ où R4 est un groupe alkyle en $C_6$ à $C_{20}$ et m est un nombre supérieur à 15.

10. Agent selon l'une des revendications 1 à 9, **caractérisé en ce que** le co-activateur de blanchiment et/ou un sel physiologiquement acceptable de celui-ci est présent dans une quantité de 0,01 à 10%, en particulier de 0,1 à 5% en poids, par rapport au poids total de l'agent prêt à l'emploi.

11. Composition selon l'une des revendications 1 à 10, **caractérisé en ce qu'**il a une valeur de pH de 7 à 11.

12. Agent selon l'une des revendications 1 à 11, **caractérisé en ce qu'**il contient en outre au moins un sel minéral de persulfate ou de peroxodisulfate, en particulier le peroxodisulfate d'ammonium, le peroxodisulfate de potassium et/ou le peroxodisulfate de sodium.

13. Procédé d'éclaircissement de fibres de kératine, **caractérisé en ce qu'**un agent selon l'une des revendications 1 à 12, est appliqué sur les fibres de kératine, laissés pendant 5 à 60 minutes sur les fibres, puis à nouveau rincé ou lavé avec un shampooing.

14. Utilisation cosmétique d'un agent selon l'une des revendications 1 à 12, pour éclaircir des fibres de kératine, en particulier des cheveux humains.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10148845 A1
- DE 10261656 A1
- EP 1905418 A2


### In der Beschreibung aufgeführte Nicht-Patentliteratur


- **KH. SCHRADER.** Grundlagen und Rezepturen der Kosmetika. Verlag, 1989
- Kosmetik. Georg Thieme Verlag, 1995
- Römpp-Lexikon Chemie. Georg Thieme Verlag, 1997
- **KH. SCHRADER.** Grundlagen und Rezepturen der Kosmetika. Hüthig Buch Verlag, 1989